(19) Europäisches Patentamt
European Patent Office
Office européen des brevets

(11) **EP 4 772 639 A1**

(12) **EUROPEAN PATENT APPLICATION**
published in accordance with Art. 153(4) EPC

(43) Date of publication:
08.07.2026 Bulletin 2026/28

(21) Application number: 24859556.3

(22) Date of filing: 21.08.2024

(51) International Patent Classification (IPC):
C12N 15/55 (2006.01)    C08J 11/24 (2006.01)
C08J 11/26 (2006.01)    C12N 1/15 (2006.01)
C12N 1/19 (2006.01)    C12N 1/21 (2006.01)
C12N 5/10 (2006.01)    C12N 9/16 (2006.01)
C12N 15/63 (2006.01)    C12P 7/18 (2006.01)
C12P 7/40 (2006.01)    C12P 7/62 (2022.01)

(52) Cooperative Patent Classification (CPC):
C08J 11/24; C08J 11/26; C12N 5/10; C12N 9/14;
C12N 9/16; C12N 15/63; C12N 15/74; C12N 15/80;
C12N 15/81; C12P 7/18; C12P 7/40; C12P 7/62;
Y02W 30/62

(86) International application number:
PCT/JP2024/029558

(87) International publication number:
WO 2025/047523 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.08.2023 JP 2023139358

(71) Applicants:
• Bell Polyester Products, Inc.
Yamaguchi 747-0823 (JP)
• National University Corporation Nara Institute of
Science and Technology
Ikoma-shi, Nara 630-0192 (JP)

(72) Inventors:
• IWASAKI Mai
Houfu-shi, Yamaguchi 747-0823 (JP)

• KATSUMA Keita
Houfu-shi, Yamaguchi 747-0823 (JP)
• OKIMOTO Masaya
Houfu-shi, Yamaguchi 747-0823 (JP)
• ASAMURA Naoya
Houfu-shi, Yamaguchi 747-0823 (JP)
• YOSHIDA Shosuke
Ikoma-shi, Nara 630-0192 (JP)

(74) Representative: Henkel & Partner mbB
Patentanwaltskanzlei, Rechtsanwaltskanzlei
Maximiliansplatz 21
80333 München (DE)

Remarks:
The complete document including Reference
Table(s) and the Sequence Listing(s) can be
downloaded from the EPO website

(54) **ESTERASE, POLYESTER DECOMPOSITION PRODUCT, POLYESTER TREATMENT METHOD, AND POLYESTER PRODUCTION METHOD**

(57) The present invention addresses the problem of obtaining an esterase having a high decomposition activity at a low temperature. In order to solve this problem, provided is an esterase which has at least 80% sequence identity with the amino acid sequence represented by SEQ ID NO: 1 and in which A172 and A209 in the amino acid sequence are substituted.

EP 4 772 639 A1

[FIG. 1]

## Description

Cross Reference to Related Applications

The present application is based upon and claims the benefit of the priority of Japanese Patent Application No. 2023-139358 (filed on August 29, 2023), the entire of which is incorporated herein in its entirety by reference.

Technical Field

The present disclosure relates to an enzyme (esterase) that hydrolyzes an ester compound into acid and alcohol. The present disclosure relates to a method for treating a polyester with an esterase. Moreover, the present disclosure relates to a method for producing a polyester using an esterase. The present disclosure relates to a polynucleotide, a vector and a host cell for producing an esterase.

Background Art

A polyester resin such as polyethylene terephthalate (PET), which is a general plastic, is one of the plastic materials for which a recycling system has been established the most. However, in the current recycling systems, so-called one-way recycling is the mainstream. One-way recycling refers to a system in which plastic products are reused as a raw material for other products, without being reused in their original shapes after use. For example, a PET bottle, one of the applications of the polyester resin, undergo steps of washing, melting, and remolding after use, and changes its shape into fibers for clothing and sheets for industrial applications to be used. Such recycling is called as material recycling. In material recycling, the quality of reused plastic products deteriorates by each recycling, so that a circulation-type recycling system that circulates plastic resources cannot be established.

Therefore, chemical recycling is being investigated as a recycling method that enables circulating recycling system for plastic resources. Chemical recycling is to chemically decompose waste plastics, and reuse decomposition products as raw materials. Since plastics are decomposed to a compound level in chemical recycling, the quality of reused products can be stabilized, and the decomposition products can be used for raw materials of products other than plastics; therefore, it is a useful recycling method for establishing the resource circulating recycling system. However, chemical recycling utilizing thermal decomposition, for example, requires depolymerization of plastics at high temperature and purification of depolymerization products by distillation, so that significant heat energy is required for recycling.

Therefore, chemical recycling that decomposes a polyester with an enzyme has been attracting attention recently (for example, refer to Patent Literature 1 and Patent Literature 2). As an enzyme capable of decomposing a polyester, a cutinase-like esterase is described in Patent Literature 1. In Patent Literature 2, a mutant of the esterase described in Patent Literature 1 is described.

Citation List

Patent Literature

Patent Literature 1: International Publication No. WO2012/099018A
Patent Literature 2: Japanese Translation of PCT International Application Publication No. 2019-520833A

Non-Patent Literature

Non-Patent Literature 1: Turnier et al., "An engineered PET depolymerase to break down and recycle plastic bottles" Nature, Vol. 580, p.p. 216-219 (2020)
Non-Patent Literature 2: Shosuke Yoshida et al., "A bacterium that degrades and assimilates poly(ethylene terephthalate)" Sciencemag.org, Vol. 351, p.p. 1196 (2016)

Summary of Invention

Technical Problem

According to the esterase described in Patent Literature 1, even under the condition having the maximum decomposition activity, it decomposed only about 30% in one week and only about 54% in two weeks, and the decomposition rate is not sufficient in an industrial level.

To increase the decomposition rate of the polyester by the enzyme, it seems that heat resistance of the enzyme may be increased to decompose the polyester under a high-temperature condition. However, if the polyester is left in a temperature environment close to the glass transition temperature, crystallization of the polyester proceeds. For example, Non-Patent Literature 1 indicates that crystallization of polyethylene terephthalate proceeds at 70°C which is lower than its glass transition temperature, which is approximately 75°C. In general, the rate of enzymatic decomposition of the polyester having a high crystallinity is significantly lower than the rate of enzymatic decomposition of the polyester having a low crystalinity (for example, refer to Non-Patent Literature 2).

Therefore, there is a demand for an esterase having a high decomposition activity at a temperature lower than the temperature at which crystalization of a polyester, which is a decomposition target, proceeds.

In addition, from the viewpoint of energy costs, there is a demand for an esterase having a high decomposition activity at lower temperature.

Solution to Problem

According to a first aspect of the present disclosure, an esterase is provided, the esterase which has at least 80% sequence identity with an amino acid sequence shown in SEQ ID NO: 1, and in which A172 and A209 in the amino acid sequence are substituted.

According to a second aspect of the present disclosure, a method for treating a polyester is provided, the method comprising a first step of decomposing a polyester by using the esterase according to the first aspect.

According to a third aspect of the present disclosure, a method for producing a polyester is provided, the method comprising a fourth step of producing a polyester using a compound isolated in the method for treating a polyester according to the second aspect as at least a part of a monomer.

According to a fourth aspect of the present disclosure, a polyester decomposition product acquired by decomposing a polyester with the esterase according to the first aspect is provided.

According to a fifth aspect of the present disclosure, a method for producing a polyester by using the polyester decomposition product according to the fourth aspect is provided.

According to a sixth aspect of the present disclosure, a polynucleotide encoding the esterase according to the first aspect is provided.

According to a seventh aspect of the present disclosure, a vector including the polynucleotide according to the sixth aspect is provided.

According to an eighth aspect of the present disclosure, a host cell to which the polynucleotide according to the sixth aspect is introduced is provided.

Advantageous Effects of Invention

According to the esterase and the method for treating the polyester of the present disclosure, the polyester can be enzymatically decomposed into acid and alcohol at a high decomposition rate.

According to the esterase and the method for treating the polyester of the present disclosure, the polyester can be decomposed under an environment in which progress of crystallization of the polyester can be suppressed.

According to the esterase and the method for treating the polyester of the present disclosure, energy costs according to decomposition treatment of the polyester can be suppressed.

The esterase of the present disclosure has low substrate specificity.

According to the method for producing the polyester of the present disclosure, a high-quality recycled polyester can be produced.

According to the method for producing the polyester of the present disclosure, resource circulating recycling of the polyester can be achieved.

Brief Description of Drawings

[Fig. 1] Figure 1 is a graph showing a recovery volume of terephthalic acid in Test examples 1, 3, 5 and 7.
[Fig. 2] Figure 2 is a graph showing a rate of increase in the recovery volume of terephthalic acid in Test examples 1, 3, 5, and 7.

[Fig. 3] Figure 3 is a graph showing a recovery volume of isophthalic acid in Test examples 1, 3, 5 and 7.

[Fig. 4] Figure 4 is a graph showing a rate of increase in the recovery volume of isophthalic acid in Test examples 1, 3, 5, and 7.

[Fig. 5] Figure 5 is a graph showing a recovery volume of all components in Test examples 1, 3, 5 and 7.

[Fig. 6] Figure 6 is a graph showing a rate of increase in the recovery volume of all components in Test examples 1, 3, 5, and 7.

[Fig. 7] Figure 7 is a graph showing a recovery volume of terephthalic acid in Test examples 2, 4, 6 and 8.

[Fig. 8] Figure 8 is a graph showing a rate of increase in the recovery volume of terephthalic acid in Test examples 2, 4, 6 and 8.

[Fig. 9] Figure 9 is a graph showing a recovery volume of isophthalic acid in Test examples 2, 4, 6 and 8.

[Fig. 10] Figure 10 is a graph showing a recovery volume of isophthalic acid in Test examples 2, 4, 6 and 8.

[Fig. 11] Figure 11 is a graph showing a recovery volume of all components in Test examples 2, 4, 6 and 8.

[Fig. 12] Figure 12 is a graph showing a rate of increase in the recovery volume of all components in Test examples 2, 4, 6 and 8.

[Fig. 13] Figure 13 is a graph showing transition of a recovery volume of a decomposition product in Test example 9.

[Fig. 14] Figure 14 is a graph showing transition of a recovery volume of a decomposition product in Test example 10.

[Fig. 15] Figure 15 is a graph showing transition of a recovery volume of a decomposition product in Test example 11.

[Fig. 16] Figure 16 is a graph showing transition of a recovery volume of a decomposition product in Test example 12.

[Fig. 17] Figure 17 is a graph showing a recovery volume of a decomposition product in Test example 13.

[Fig. 18] Figure 18 is a graph showing a recovery volume of a decomposition product in Test example 14.

Description of Embodiments

According to a preferred mode of the above aspect, A172 and A209 are substituted with a cysteine residue (C).

According to a preferred mode of the above aspect, D203 and S248 in the amino acid sequence are further substituted.

According to a preferred mode of the above aspect, D203 and S248 are substituted with a cysteine residue (C).

According to a preferred mode of the above aspect, D203C and S248C form a disulfide bond.

According to a preferred mode of the above aspect, in the first step, the polyester is decomposed in a treatment solvent having a temperature of 40°C or greater and a temperature lower than the glass transition temperature of the polyester within a range of pH 7 to 10.

According to a preferred mode of the above aspect, in the first step, the polyester is decomposed in the treatment solvent having a temperature of 65°C or lower.

According to a preferred mode of the above aspect, the polyester includes 50 mol% or greater of a terephthalic acid component with respect to the total amount of an acid component, and 65 mol% or greater of an ethylene glycol component with respect to the total amount of an alcohol component.

According to a preferred mode of the above aspect, the polyester further includes 2 mol% or greater of an isophthalic acid component with respect to the total amount the acid component.

According to a preferred mode of the above aspect, the polyester is at least either of a post-consumer product and a pre-consumer product.

According to a preferred mode of the above aspect, the method for treating the polyester comprises a second step of isolating at least one compound selected from a group consisting of terephthalic acid, mono(2-hydroxyethyl) terephthalate, bis(2-hydroxyethyl) terephthalate, and salts thereof from the decomposition product acquired in the first step.

According to a preferred mode of the above aspect, in the second step, at least one compound selected from a group consisting of isophthalic acid, mono(2-hydroxyethyl) isophthalate, bis(2-hydroxyethyl) isophthalate, and salts thereof is isolated together.

According to a preferred mode of the above aspect, the method for treating the polyester further comprises a third step of isolating a decomposition product derived from the alcohol component of the polyester from the decomposition product acquired in the first step.

According to a preferred mode of the above aspect, in the method for producing the polyester, the compound includes at least one compound selected from a group consisting of terephthalic acid, mono(2-hydroxyethyl) terephthalate, and bis(2-hydroxyethyl) terephthalate in the fourth step.

According to a preferred mode of the above aspect, in the method for producing the polyester, the compound includes at least one compound selected from a group consisting of isophthalic acid, mono(2-hydroxyethyl) isophthalate, and bis(2-hydroxyethyl) isophthalate in the fourth step.

According to a preferred mode of the above aspect, in the method for producing the polyester, the compound includes ethylene glycol in the fourth step.

In the present disclosure, a substitution position of the amino acid is based on an amino acid number shown in SEQ ID NO: 1.

In the present disclosure, "sequence identity" is a parameter that shows relevance of two amino acid sequences, and shows a degree of indentity of two amino acid sequences. Identity can be calculated with a homology search program BLAST (Basic Local Alignment Search Tool). For example, a website provided by National Center for Biotechnology Information (https://blast.ncbi.nlm.nih.gov/Blast.cgi?PAGE=Proteins) can be used to calculate sequence identity.

In the present disclosure, "A172" indicates an alanine residue at 172th position in the amino acid sequence shown in SEQ ID NO: 1, for example. "A172C" indicates that the alanine residue at 172th position is substituted with a cysteine residue.

In the present disclosure, "substitution" related to the amino acid sequence indicates that an amino acid residue is substituted with another amino acid residue in the amino acid sequence.

In the present disclosure, the amino acid sequence can be determined by using the Edman degradation, or mass spectrometry (e.g., MS/MS method), for example.

The esterase of the present disclosure may include a disulfide bond.

<First embodiment>

An esterase according to a first embodiment of the present disclosure is described.

The esterase according to the first embodiment has at least 80% sequence identity with an amino acide sequence shown in SEQ ID NO: 1. The amino acid sequence shown in SEQ ID NO: 1 is an amino acid sequence of an esterase (LCC; leaf-branch compost cutinase) described in International Publication No. WO2012/099018A (Patent Literature 1). The esterase according to the first embodiment has preferably 85% or greater, and more preferably 90% or greater sequence identity with the amino acid sequence shown in SEQ ID NO: 1.

In the esterase according to the first embodiment, A172 and A209 in the amino acid sequence shown in SEQ ID NO: 1 are substituted.

In the esterase according to the first embodiment, A172 is preferably substituted with a cysteine residue (C).

In the esterase according to the first embodiment, A209 is preferably substituted with a cysteine residue (C).

In the esterase according to the first embodiment, A172 and A209 are preferably substituted with a cysteine residue (C), respectively.

The esterase according to the first embodiment can be produced by a publicly known method. For example, the esterase according to the first embodiment can be produced by a genetic engineering technique. For example, a vector including a polynucleotide encoding the esterase according to the first embodiment is introduced to a microorganism and/or a host cell and proliferated. Escherichia coli, for example, can be used as the host cell. The esterase expressed in the microorganism and/or the host cell is collected and purified to acquire the esterase.

The esterase according to the first embodiment can preferably decompose the polyester as explained in a method for treating a polyester, which will be described later. For example, the esterase according to the first embodiment can have a high activity at a temperature lower than the glass transition temperature of the polyester in which a terephthalic acid component and an ethylene glycol component configure its main skeleton. The esterase according to the first embodiment can efficiently decompose the polyester at 65°C or lower, for example, preferably 60°C or lower, and more preferably at a temperature ranging from 50°C to 60°C. Moreover, the esterase according to the first embodiment has an activity under an environment of 40°C or greater, preferably 45°C or greater, and more preferably 50°C or greater, so that the polyester can be decomposed more efficiently.

According to the esterase according to the first embodiment, the polyester can be decomposed at a temperature of which progress of crystallization of the polyester is suppressed. Accordingly, slowing down of the decomposition rate can be suppressed.

According to the esterase according to the first embodiment, the decomposition temperature can be decreased, so that energy costs can be reduced.

The esterase according to the first embodiment has low substrate specificity, and the substrate is not limited to polyethylene terephthalate. For example, the esterase according to the first embodiment can decompose not only a homopolymer of polyethylene terephthalate, but also a polyester in which a component other than a terephthalic acid component and an ethyleneglycol component (e.g., isophthalic acid) are copolymerized with polyethylene terephthalate with high efficiency

<Second embodiment>

An esterase according to a second embodiment of the present disclosure is described.

In the esterase according to the second embodiment, it is preferred that at least one selected from a group consisting of D203 and S248 in the amino acid sequence in the esterase according to the first embodiment is further substituted, and it is more preferred that D203 and S248 are further substituted.

In the esterase according to the second embodiment, at least one selected from a group consisting of D203 and S248 is preferably substituted with a cysteine residue (C), and D203 and S248 are more preferably substituted with a cysteine residue (C).

D203C and S248C further preferably form a disulfide bond. Formation of the disulfide bond can be confirmed with electrophoresis after reduction.

The esterase according to the second embodiment can be produced with the same method as the esterase according to the first embodiment.

The esterase according to the second embodiment can have the same effect as the esterase according to the first embodiment.

The esterase according to the second embodiment has a higher heat resistance than the esterase according to the first embodiment. The esterase according to the second embodiment can have a higher decomposition activity than the esterase according to the first embodiment at a temperature closer to the glass transition temperature. The esterase according to the second embodiment, for example, can efficiently decompose the polyester at a temperature ranging from 55°C to 65°C. Therefore, by making the decomposition environment closer to the glass transition temperature, the esterase according to the second embodiment can decompose the polyester more efficiently than the esterase according to the first embodiment.

<Third embodiment>

A method for treating the polyester according to a third embodiment is described. The method for treating the polyester according to the third embodiment of the present disclosure is also a method for producing a monomer and/or an oligomer of the polyester.

The method for treating the polyester according to the third embodiment comprises a first step of decomposing the polyester by using at least one selected from a group consisting of the esterase according to the first embodiment and the esterase according to the second embodiment.

Water, for example, can be used as a treatment solvent.

The concentration of the esterase in the treatment solvent may be 30 nM or greater, 50 nM or greater, 80 nM or greater, 100 nM or greater, or 200 nM or greater, for example. The concentration of the esterase may be 1000 nM or lower, 500 nM or lower, or 200 nM or lower.

The esterase may be $0.5 \times 10^{-9}$ mol or greater, $1 \times 10^{-9}$ mol or greater, $0.5 \times 10^{-8}$ mol or greater, $1 \times 10^{-8}$ mol or greater, $0.5 \times 10^{-7}$ mol or greater, or $1 \times 10^{-7}$ mol or greater, with respect to 1 g of the polyester, which is the decomposition target, for example. The esterase may be $1 \times 10^{-6}$ mol or lower, $0.5 \times 10^{-6}$ mol or lower, $1 \times 10^{-7}$ mol or lower, $0.5 \times 10^{-7}$ mol or lower, $1 \times 10^{-8}$ mol or lower, or $0.5 \times 10^{-8}$ mol or lower, with respect to 1 g of the polyester, which is the decomposition target.

The decomposition temperature, i.e., the temperature of the treatment solvent, may be 40°C or greater, preferably 45°C or greater, and more preferably 50°C or greater. By increasing the decomposition temperature, the decomposition rate can be increased. The decomposition temperature is preferably lower than the glass transition temperature of the polyester, which is the decomposition target. When the decomposition temperature is equal to or greater than the glass transition temperature, crystallization of the polyester proceeds during treatment, and the decomposition rate decreases. The decomposition temperature may be lower than 70°C, 65°C or lower, 60°C or lower, or 55°C or lower, for example.

The pH of the treatment solvent may be within a range of 7 to 11, and more preferably within a range of 7 to 10. Since the pH decreases during decomposition, the pH is preferably maintained (e.g., maintained at pH 9) using alkali (e.g., sodium hydroxide aqueous solution).

The crystallinity of the polyester, which is the decomposition target, is 7% or lower, preferably 6% or lower, more preferably 5% or lower, and further preferably 3% or lower. The polyester is preferably amorphous.

Using a Differential Scanning Calorimetry (DSC; Differential Scanning Calorimetry) apparatus, the temperature of the polyester is increased from 30°C to 300°C at a heating rate of 10°C/min in nitrogen to measure the crystallization temperature (Tc), the crystallization enthalpy (ΔHc), the melting point (Tm), and the quantity of heat of fusion (ΔHm), and the crystallinity can be calculated by the following equation.

$$\text{Crystallinity (\%)} = ((\Delta Hm - \Delta Hc) / \Delta Hm) \times 100$$

In the polyester, which is the decomposition target, the acid component can include 50 mol% or greater of a terephthalic acid component. For example, the terephthalic acid component may be 55 mol% or greater, 60 mol% or greater, 65 mol% or greater, 70 mol% or greater, 75 mol% or greater, 80 mol% or greater, 85 mol% or greater, 90 mol% or greater, 95 mol% or greater, or 100 mol%, with respect to the total amount of the acid component. For example, the terephthalic acid component may be 98 mol% or lower, 95 mol% or lower, 90 mol% or lower, 85 mol% or lower, 80 mol% or lower, 75 mol% or lower, 70 mol% or lower, or 65 mol% or lower, with respect to the total amount of the acid component.

In the polyester, which is the decomposition target, the acid component can further include 2 mol% or greater of an isophthalic acid component with respect to the acid component. When the acid component includes the isophthalic acid component as a copolymerization component, the isophthalic acid component may be, for example, 5 mol% or greater, 10 mol % or greater, 15 mol% or greater, or 20 mol% or greater, with respect to the total amount of the acid component. The isophthalic acid component may be, for example, 25 mol% or lower, 20 mol% or lower, 15 mol% or lower, 10 mol% or lower, or 5 mol% or lower, with respect to the total amount of the acid component. The acid component may be consisted of the terephthalic acid component and the isophthalic acid component (100 mol% in total).

In the polyester, which is the decomposition target, the acid component may include other acid components. Examples of other acid components include orthophthalic acid, 2,6-naphthalenedicarboxylic acid, 2,4-furandicarboxylic acid, 2,5-furandicarboxylic acid, adipic acid, sebacic acid, succicnic acid, dimer acid, 1,4-cyclohexanedicarboxylic acid, dimethyl terephthalate, and dimethyl isophthalate. These other acid components may be added singly, or two or more types may be added at any proportion.

In the polyester, which is the decomposition target, the alcohol component may include 65 mol% or greater of an ethylene glycol component. For example, the ethylene glycol component may be 70 mol% or greater, 75 mol% or greater, 80 mol% or greater, 85 mol% or greater, 90 mol% or greater, 95 mol% or greater, or 100 mol%, with respect to the total amount of the alcohol component. For example, the ethylene glycol component may be 95 mol% or lower, 90 mol% or lower, 85 mol% or lower, 80 mol% or lower, or 75 mol% or lower, with respect to the total amount of the alcohol component. The alcohol component may be consisted of the ethylene glycol component (100 mol% in total).

In the polyester, which is the decomposition target, the alcohol component may include other alcohol components. Examples of other alcohol components include neopentyl glycol (2,2-dimethyl-1,3-propanediol), 1,3-propanediol, 2-methyl-1,3-propanediol, 2-butyl-2-ethyl-1,3-propanediol, 1,2-propanediol, 1,4-butanediol, 1,3-butanediol, diethylene glycol, 1,4-cyclohexanediol, and 1,4-cyclohexanedimethanol. These other alcohol components may be added singly, or two or more types may be added at any proportion.

The polyester, which is the decomposition target, may be polyethylene terephthalate (PET; polyethylene terephthalate), and a polyester that has polyethylene terephthalate as a main skeleton, for example, and in which the acid component has the isophthalic acid component. The polyester, which is the decomposition target, may be a mixture (blend, polymer alloy) of multiple polyesters. Moreover, the polyester, which is the decomposition target, may be a laminate (laminated film) of multiple polyesters.

A monomer component of the polyester, which is the decomposition target, may be a petroleum-derived component, a biomass-derived component, or a component derived from a recycled material.

The biomass-derived component can be detected by measuring the number of radioactive carbon with Accelerator Mass Spectrometry (AMS; Accelerator Mass spectrometry), for example. The amount of $^{14}C$ and $^{13}C$ that are isomers of $^{12}C$ can be measured by AMS measurement, and usage of the biomass-derived raw material can be measured from the amount of $^{14}C$ and $^{13}C$.

The polyester, which is the decomposition target, may be at least either of a post-cosumer product and a pre-consumer product.

The post-consumer product indicates a used polyester product. The used polyester product is not particularly limited, and examples thereof include those that are collected after being used as products such as cosmetic containers, bottles, and sheets.

The pre-consumer product indicates a polyester that is not handled as a product, and it is not particularly limited. Examples thereof include trashes generated during steps of producing polyester products, nonconforming products, oligomers generated during a polymerization step, and scraps and trashes generated at molding.

The shape of the polyester, which is the decomposition target, is not particularly limited, and it may be processed into forms of powders, flakes, sheets, films, and pellets as necessary. They may be used alone, or used as a mixture.

The method for treating the polyester further comprises a second step of isolating a decomposition product of the polyester from the decomposition product acquired in the first step. The decomposition product includes a mixture of a monomer and/or an oligomer of the polyester.

In the second step, when the polyester includes the terephthalic acid component and the ethylene glycol component, at least one compound selected from a group consisting of terephthalic acid, mono(2-hydroxyethyl) terephthalate, bis(2-hydroxyethyl) terephthalate, and salts thereof is preferably isolated as the decomposition product of the polyester.

In the second step, when the polyester includes the terephthalic acid component, the isophthalic acid component and the ethylene glycol component, at least one compound selected from a group consisting of terephthalic acid, mono(2-hydroxyethyl) terephthalate, bis(2-hydroxyethyl) terephthalate, and salts thereof is preferably isolated as the decomposition product of the polyester, and it is more preferred that at least one compound selected from a group consisting of isophthalic acid, mono(2-hydroxyethyl) isophthalate, bis(2-hydroxyethyl) isophthalate, and salts thereof is further isolated together.

Among the decomposition product, a water-insoluble compound derived from the acid component of the polyester can be separated from the treatment solvent and collected by making the treatment solvent acidic, for example. The acid used for neutralizaton may be sulfuric acid or hydrochloric acid, for example. The acid component of the decomposition product may perform steps of distillation and liquid separation. When the polyester includes the terephthalic acid component and the ethylene glycol component, examples of the compound including the acid component of the decomposition product include at least one compound selected from a group consisting of terephthalic acid, mono(2-hydroxyethyl) terephthalate, and bis(2-hydroxyethyl) terephthalate. When the polyester includes isophthalic acid component and the ethylene glycol component, examples of the water-insoluble compound derived from the acid component include at least one compound selected from a group consisting of isophthalic acid, mono(2-hydroxyethyl) isophthalate, and bis(2-hydroxyethyl) isophthalate.

It is preferred that the method for treating the polyester further comprises a third step of isolating the decomposition product derived from the alcohol component of the polyester from the decomposition product acquired in the first step.

Examples of the compound isolated in the third step includes ethylene glycol, neopentyl glycol (2,2-dimethyl-1,3-propanediol), 1,3-propanediol, 2-methyl-1,3-propanediol, 2-butyl-2-ethyl-1,3-propanediol, 1,2-propanediol, 1,4-butanediol, 1,3-butanediol, diethylene glycol, 1,4-cyclohexanediol, 1,4-cyclohexanedimethanol, mono(2-hydroxyethyl) terephthalate, and bis(2-hydroxyethyl) terephthalate.

In the third step, when the polyester includes the ethylene glycol component, the decomposition product derived from the alcohol component of the polyester to be isolated preferably includes ethylene glycol, and may be only ethylene glycol.

Among the decomposition product, the alcohol component of the decomposition product of the polyester can be separated from the treatment solvent by distillation, for example. When the polyester includes the ethylene glycol component, examples of the compound including the alcohol component of the decomposition product include at least one compound selected from a group consisting of ethylene glycol, diethylene glycol, mono(2-hydroxyethyl) terephthalate, and bis(2-hydroxyethyl) terephthalate.

When a single compound cannot be isolated from the acid component and the alcohol component separated from the treatment solvent or when isolation is unnecessary, it can be collected as a mixture of decomposition products. The content proportion of each component in the mixture can be analyzed by Nuclear Magnetic Resonance (NMR; Nuclear Magnet Resonance). The decomposition product can be applied to the method for producing the polyester according to the fourth embodiment as a mixture.

According to the method for treating the polyester according to the third embodiment, the polyester can be decomposed with high efficiency. Moreover, the decomposition product acquired by the method for treating the polyester according to the third embodiment can be used as a raw material. For example, the decomposition product can be used to reproduce a new polyester.

According to the method for treating the polyester according to the third embodiment, the decomposition temperature can be decreased, so that the polyester can be decomposed with reduced energy costs.

According to the method for treating the polyester according to the third embodiment, not only homopolymers of polyethylene terephthalate, but also polyesters in which a component other than the terephthalic acid component and the ethylene glycol component (e.g., isophthalic acid) is copolymerized with polyethylene terephthalate can be decomposed with high efficiency.

<Fourth embodiment>

A method for producing the polyester according to a fourth embodiment of the present disclosure is described.

The method for producing the polyester according to the fourth embodiment of the present disclosure comprises a fourth step of producing the polyester by using the decomposition product in the method for treating the polyester according to the third embodiment as at least a part of a monomer. Examples of the decomposition product include the above-described compounds isolated in the third embodiment.

A raw material monomer other than the decomposition product in the method for producing the polyester of the present disclosure can be suitably adjusted depending on the properties of the target polyester, and is not particularly limited.

In the method for producing the polyester of the present disclosure, the raw material monomer other than the decomposition product may be used or may not be used. When the raw material monomer other than the decomposition product is used, it may be a petroleum-derived component, a biomass-derived component, or a component derived from a recycled material

Examples of an acid component of the raw material monomer other than the decomposition product in the method for producing the polyester of the present disclosure include terephthalic acid, isophthalic acid, orthophthalic acid, 2,6-naphthalenedicarboxylic acid, 2,4-furandicarboxylic acid, 2,5-furandicarboxylic acid, adipic acid, sebacic acid, succicnic acid, dimer acid, 1,4-cyclohexanedicarboxylic acid, dimethyl terephthalate, dimethyl isophthalate, bis-2-hydroxyethyl terephthalate, and bis-2-hydroxyethyl isophthalate. These acid components may be added singly, or two or more types may be added at any proportion.

Examples of an alcohol component of the raw material monomer other than the decomposition product in the method for producing the polyester of the present disclosure include ethylene glycol, neopentyl glycol (2,2-dimethyl-1,3-propanediol), 1,3-propanediol, 2-methyl-1,3-propanediol, 2-butyl-2-ethyl-1,3-propanediol, 1,2-propanediol, 1,4-butanediol, 1,3-butanediol, diethylene glycol, triethylene glycol, 1,4-cyclohexanediol, 1,4-cyclohexanedimethanol, polyethylene glycol, polytrimethylene glycol, and polytetramethylene glycol. These alcohol components may be added singly, or two or more types may be added at any proportion.

The blending ratio of the decomposition product in the method for producing the polyester of the present disclosure is acquired by dividing the mass of the component derived from the decomposition product in the recycled polyeter by the mass of the total recycled polyester. For example, the blending ratio of the decomposition product may be 10% by mass or greater, 20% by mass or greater, 30% by mass or greater, 50% by mass or greater, 70% by mass or greater, 90% by mass or greater, 95% by mass or greater, or 100% by mass. For example, the blending ratio of the decomposition product may be 95% by mass or lower, 90% by mass or lower, 70% by mass or lower, 50% by mass or lower, 30% by mass or lower, or 20% by mass or lower.

The blending ratio of the component derived from a recycled material including the decomposition product in the method for producing the polyester of the present disclosure is acquired by adding the mass of the component derived from the decomposition product in the recycled polyester and the mass of the component derived from a recycled material among the monomer component other than the decomposition product in the recycled polyester, and dividing the sum by the mass of the total recycled polyester. For example, the blending ratio of the component derived from a recycled material including the decomposition product may be 10% by mass or greater, 20% by mass or greater, 30% by mass or greater, 50% by mass or greater, 70% by mass or greater, 90% by mass or greater, 95% by mass or greater, or 100% by mass. For example, the blending ratio of the component derived from a recycled material including the decompositon product may be 95% by mass or lower, 90% by mass or lower, 70% by mass or lower, 50% by mass or lower, 30% by mass or lower, or 20% by mass or lower.

A polymerization step in the method for producing the polyester of the present disclosure is not particularly limited, and can be performed by a publicly known polymerization method using a publicly known catalyst. The method for producing the polyester may be either of a method of directly esterifying unsubstituted polycarboxylic acid as a starting raw material, or a method of performing transesterification using an esterified product such as dimethyl ester as a starting raw material. Upon producing the polyester, it is desired to perform a transesterification by using a publicly known catalyst under normal pressure as a first stage, and to perform a polycondensation reaction by using a publicly known catalyst under reduced pressure as a following second stage, so that a sufficient reaction rate can be achieved. After the polycondensation reaction, a solid-phase polymerization reaction may be performed. Intrinsic viscosity of the polyester can be increased by the solid-phase polymerization reaction.

According to the method for producing the polyester according to the fourth embodiment of the present disclosure, the polyester can be reproduced from the decomposition product of the polyester decomposed by using the esterase.

<Fifth embodiment>

A polyester decomposition product according to a fifth embodiment of the present disclosure is described.

The polyester decomposition product according to the fifth embodiment of the present disclosure is acquired by decomposing the polyester with the esterase according to the first embodiment and/or the esterase according to the second embodiment of the present disclosure.

The polyester decomposition product according to the fifth embodiment of the present disclosure is the same as a decomposition treatment product acquired in the first step of the method for treating the polyester according to the third embodiment, and includes a mixture of a monomer and/or an oligomer of the polyester.

The polyester decomposition product according to the fifth embodiment of the present disclosure can be distinguished from conventional polyester decomposition products from the point that the esterase according to the first embodiment and/or the esterase according to the second embodiment is/are included.

The polyester can be produced by using the polyester decomposition product according to the fifth embodiment of the present disclosure. In this case, the polyester can be produced in a same manner as the method for producing the polyester according to the fourth embodiment of the present disclosure.

<Sixth embodiment>

A polyester according to a sixth embodiment of the present disclosure is described.

The polyester according to the sixth embodiment of the present disclosure is a polyester produced from the production method according to the fourth embodiment. That is, the polyester according to the sixth embodiment of the present disclosure is a polyester produced by using the decomposition product of the polyester as the raw material.

<Seventh embodiment>

A polynucleotide according to a seventh embodiment of the present disclosure encodes the esterases according to the first embodiment and the second embodiment. A vector includes the polynucleotide. Moreover, a host cell is a host cell to which the polynucleotide and/or the vector is/are introduced. Examples of the host cell include Escherichia coli.

It may be impossible or substantially not practical to directly specify the esterase of the present disclosure from its composition, structure or characteristic. In such case, the esterase of the present disclosure should be permitted to be specified by its production method.

Examples

The esterase, the method for treating the polyester, and the method for producing the polyester of the present disclosure are described with examples in the following. However, the esterase, the method for treating the polyester, and the method for producing the polyester of the present disclosure are not limited to the following examples.

[Preparation of Esterase]

A vector pET21b(+) (Novagen, Germany) was used for the expression of a cutinase LCC in Escherichia coli BL21 (DE3) CodonPlus RIPL (Agilent Technologies GmbH, Germany) and a mutant thereof.

In order to express the gene encoding the sequence obtained by removing the N-terminal signal peptide from LCC and its variants in E. coli, codon usage was optimized, and a DNA fragment was synthesized by designing a sequence with both terminal sequences added for incorporation into the NdeI and XhoI restriction enzyme sites of the pET21b (+) vector via In-Fusion cloning. The synthesized DNA fragment was incorporated into the NdeI and XhoI restriction enzyme sites of the pET21b (+) vector by In-Fusion cloning so that a 6 x His-Tag was introduced at the C-terminus. The length of the DNA fragment incorporated into the plasmid vector was confirmed by colony PCR. PCR was performed with Gotaq Green Master Mix (manufactured by Promega Corporation), and the T7 promoter (5'-CGCGAAATTAATACGACTCACTATAG GG-3') and the T7 terminator (5'-GCTAGTTATTGCTCAGCGGTGG-3') were used as primers. The gene sequence was determined by DNA sequencing using the T7 promoter and the T7 terminator as primers. The plasmid vector was introduced into E. coli BL21 (DE3) CodonPlus RIPL.

Freshly transformed E. coli BL21 (DE3) CodonPlus RIPL cells were seeded in LB medium containing ampicillin and chloramphenicol, and protein was expressed using IPTG as a protein expression inducer. Cells were collected, disrupted using an ultrasonic crusher, and centrifuged at 14,000 rpm for 20 minutes under 4°C, and the supernatant was used as the crude enzyme solution.

The crude enzyme solution was subjected to affinity chromatography using TALON resin (manufactured by TAKARA BIO INC.). SDS-PAGE was used to confirm the bands of each eluate and confirm whether the target protein is purified. The purified protein was exchanged with 50 mM $Na_2HPO_4$-HCl, pH 7.0 buffer using a PD-10 desalting column (manufactured by Cytiva). The purified protein was analyzed with a wavelength of 280 nm to calculate the concentration of the protein. The molar extinction coefficient $\varepsilon$ of the protein was calculated using the ProtParam tool (http://web.expasy. org/protparam/).

All chemical substances are of analytical grade from Sigma Company (Germany).

[Method for Treating Polyester]

(1) Decomposed polyester

Polyesters of Sample 1 to Sample 4 shown in Table 2 were treated with enzymes A to D shown in Table 1, respectively. Sample 1 is polyethylene terephthalate (PET). Samples 2 to 4 are polyesters in which isophthalic acid is copolymerized with PET as a third component. Using an FT-NMR apparatus of DPX-400 manufactured by Brucker Co. Ltd., the copolymerization rate of isophthalic acid in each sample was measured by proton NMR spectrum by dissolving each sample in a mixed solution of trifluoroacetic acid-d and deuterated chloroform, and mixing with tetramethylsilane as a standard.

(2)Crystallinity, Glass transition temperature, Crystallization temperature, and Melting point

Table 2 shows the crystallinity, the glass transition temperature, the crystallization temperature, and the melting point of each sample.

10 mg of a polyester resin was weighted, and the temperature was increased from 30°C to 300°C at a heating rate of 10°C/min in a nitrogen atmosphere using a differential scanning calorimeter DSC (manufactured by TA Instruments Corporation, DSC2500) to measure the crystallization temperature (Tc), the crystallization enthalpy ($\Delta$Hc), the melting point (Tm), and the quantity of heat of fusion ($\Delta$Hm). The crystallization temperature (Tc) was calculated from the following equation.

$$\text{Crystallinity (Tc) (\%)} = ((\Delta Hm - \Delta Hc) / \Delta Hm) \times 100$$

[Table 1]

| Emzyme A | A172C_A209C |
|---|---|
| Emzyme B | A172C_A209C_D203C_S248C |
| Emzyme C | D203C_S248C |
| Emzyme D | LCC |

[Table 2]

| | Acid component | | Alcohol component | Crystallinity | Glass transition temperature | Crystallization temperature | Melting point |
|---|---|---|---|---|---|---|---|
| | Terephthalic acid | Isophthalic acid | Ethylene glycol | | | | |
| | (mol%) | (mol%) | (mol%) | (%) | (°C) | (°C) | (°C) |
| Sample 1 | 100 | 0 | 100 | 2.4 | 75.0 | 125.4 | 255.9 |
| Sample 2 | 95 | 5 | 100 | 3.1 | 77.6 | 145.9 | 243.0 |
| Sample 3 | 91 | 9 | 100 | 3.6 | 76.7 | 152.5 | 233.0 |
| Sample 4 | 77 | 23 | 100 | Amorphous | 62.4 | - | - |

(3) Film formation

Samples 1 to 4 were formed into films having a thickness of 0.2 mm to enlarge the contact surface with the esterase, respectively.

(4) Decomposition of Polyester

After the polyester film having a thickness of 0.2 mm and a diameter of 6 mm was added to 200 mM of Bicine-NaOH (pH 9.0), the purified enzyme acquired in the above-described method was added thereto such that the final concentration becomes 100 nM, heated at 50 to 65°C, and allowed to stand still for 3 hours, 6 hours, 24 hours, or 48 hours to react.

(5) Measurement of Decomposition product

A part of the reaction solution after reaction was fractionated, diluted with a buffer solution (80% (v/v) 20mM $NaH_2PO_4$-$H_3PO_4$ (pH 2.5)/20% (v/v) DMSO), allowed to stand still for 10 minutes at 90°C, and subjectd to heat treatment. Then, a part of the supernatant was analyzed with High Performance Liquid Chromatography (HPLC; High Performance Liquid Chromatography). Terephthalic acid, mono(2-hydroxyethyl) terephthalate, bis(2-hydroxyethyl) terephthalate and isophthalic acid solution of known concentration was used as a standard solution.

The conditions of HPLC are as follows:

Apparatus: LC-2010A HT (manufactured by Shimadzu Corporation)
Column: Cosmosil 5C18-AR-II guard column, Cosmosil 5C18-AR-II column (manufactured by NACALAI TESQUE, INC.)
Mobile phase: Methanol/20 mM $NaH_2PO_4$-$H_3PO_4$ (pH 2.5)
Flow rate: 1.0 mL/min
Detection wavelength: 240 nm
Elution condition: 0 to 15 min; 25% (v/v) methanol, 15 to 25 min; methanol was changed with a concentration gradient of 25 to 100%

The peak areas of the decomposition products (terephthalic acid, mono(2-hydroxyethyl) terephthalate, bis(2-hydroxyethyl) terephthalate, and isophthalic acid) separated and detected by HPLC were compared with the peak area of the standard solution to quantify the decomposition products under each condition.

(Test examples 1 to 8)

The polyesters of Samples 1 to 4 shown in Table 2 were used as a decomposition target to perform a decomposition test using the esterases shown in Table 1. The esterase used was LCC (enzyme D) having the amino acid sequence represented by SEQ ID NO: 1, and its mutants A172C_A209C (enzyme A), A172C_A209C_D203C_S248C (enzyme B), and D203C_S248C (enzyme C). In the following table, the notations of the enzymes A to D are used. After decomposing each sample at 50°C or 60°C for 24 hours, the recovery volumes of terephthalic acid, isophthalic acid and all components (terephthalic acid, mono (2-hydroxyethyl) terephthalate, bis (2-hydroxyethyl) terephthalate, and isophthalic acid) were measured.

Table 3 to Table 10 show the recovery volume of terephthalic acid, the recovery volume of isophthalic acid, and the recovery volume of all components. The recovery volume of all components is a total amount of terephthalic acid, isophthalic acid, mono (2-hydroxyethyl) terephthalate, and bis (2-hydroxyethyl) terephthalate.

As for the recovery volume of the decomposition product by the enzyme A, the enzyme B and the enzyme C, an increase rate with respect to the recovery volume of the decomposition product by the enzyme D was calculated. The increase rate was calculated based on the following equation. The recovery volumes shown in each table are rounded to three digits. However, the increase rate shown in the tables are calculated based on the recovery volume before rounding, so that when the increase rate is calculated based on the recovery volume shown in the tables, it may not completely match with the increase rate shown in the tables.

Increase rate (%) = (Recovery volume of Decomposition product by the target esterase ($\mu$mol) - Recovery volume of Decomposition product by LCC ($\mu$mol)) / Recovery volume of Decomposition product by LCC ($\mu$mol) x 100

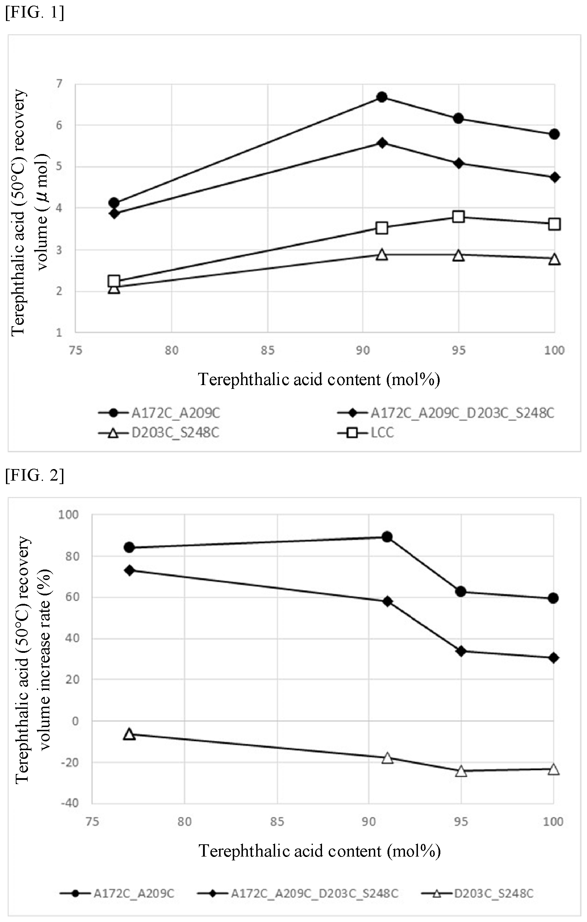

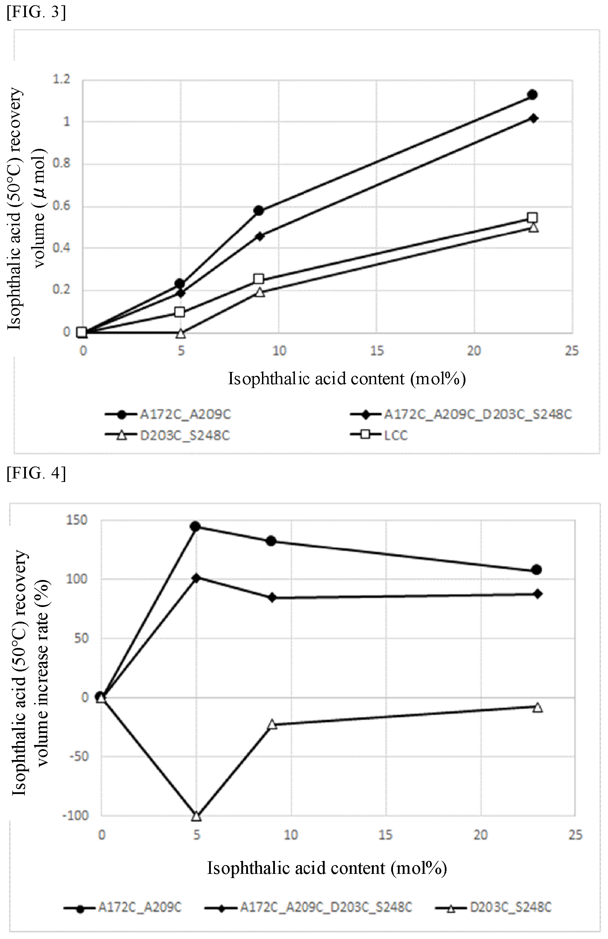

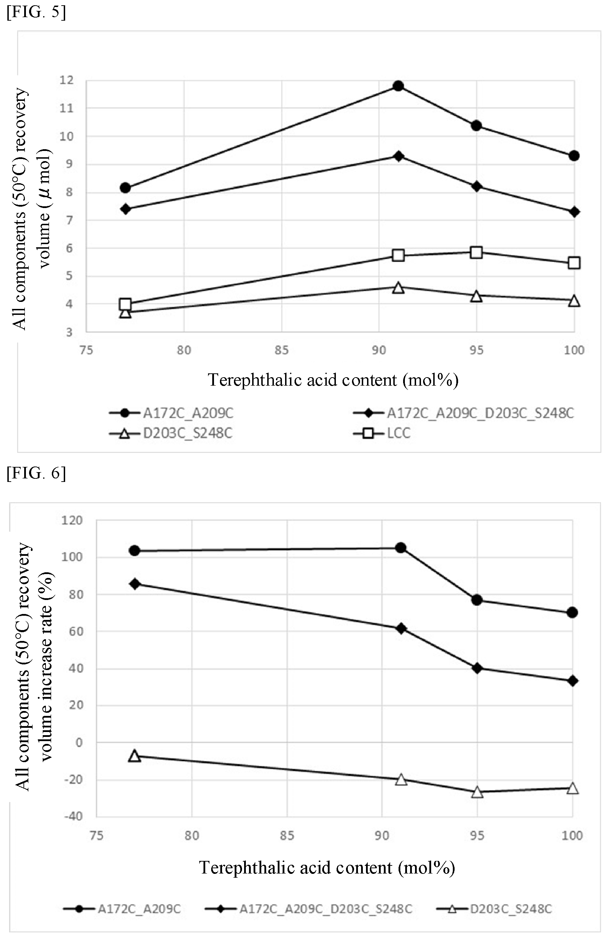

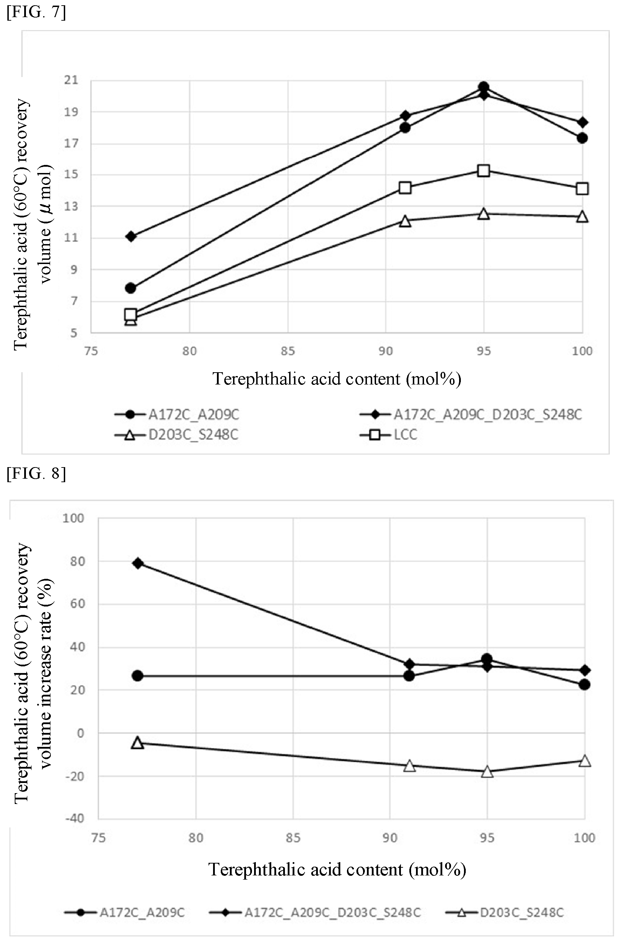

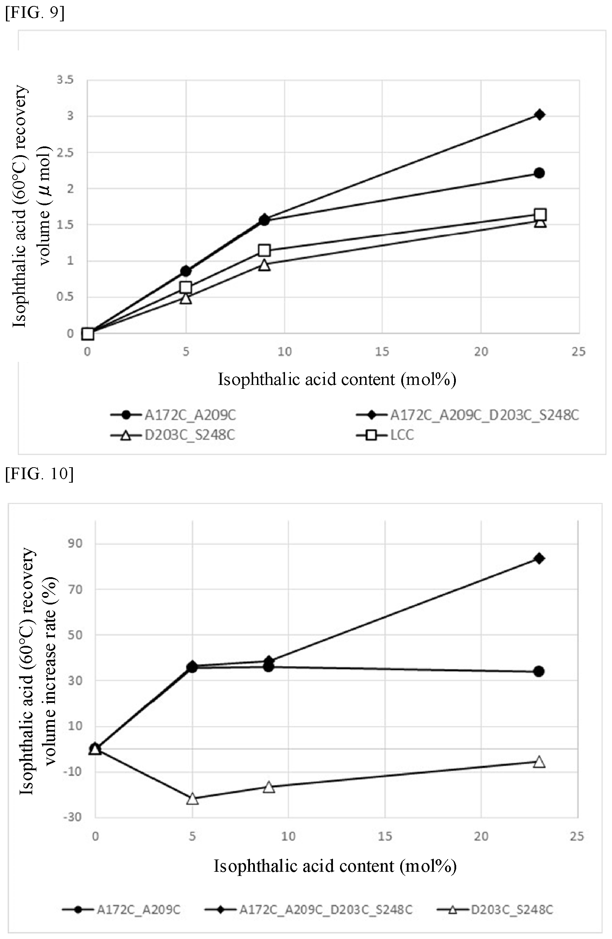

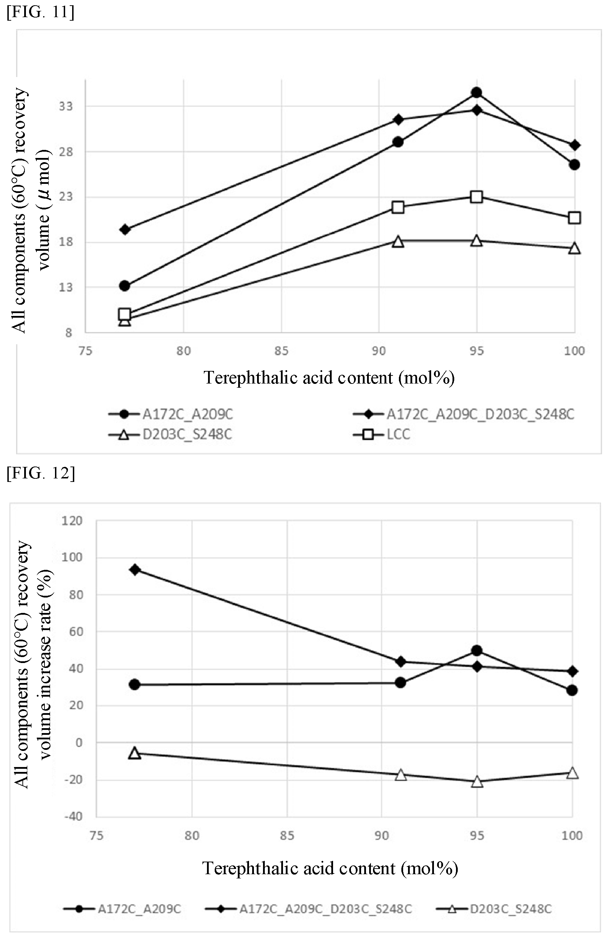

Figure 1 to Figure 8 show graphs comparing the recovery volumes of the decomposition products of Samples 1 to 4 by each esterase. Figure 1 and Figure 2 are graphs of the recovery volume of terephthalic acid when Samples 1 to 4 were decomposed at a decomposition temperature of 50°C and the increase rate thereof, respectively. Figure 3 and Figure 4 are graphs of the recovery volume of isophthalic acid when Samples 1 to 4 were decomposed at a decomposition temperature of 50°C and the increase rate thereof, respectively. Figure 5 and Figure 6 are graphs of the recovery volume of all components when Samples 1 to 4 were decomposed at a decomposition temperature of 50°C and the increase rate thereof, respectively. Figure 7 and Figure 8 are graphs of the recovery volume of terephthalic acid when Samples 1 to 4 were decomposed at a decomposition temperature of 60°C and the increase rate thereof, respectively. Figure 9 and Figure 10 are graphs of the recovery volume of isophthalic acid when Samples 1 to 4 were decomposed at a decomposition temperature of 60°C and the increase rate thereof, respectively. Figure 11 and Figure 12 are graphs of the recovery volume of all components when Samples 1 to 4 were decomposed at a decomposition temperature of 60°C and the increase rate thereof, respectively. The horizontal axes of Figures 1 to 2, Figures 5 to 8, and Figures 11 to 12 show the content of terephthalic acid included in the acid components of Samples 1 to 4. The horizontal axes of Figures 3 to 4 and Figures 9 to 10 show the content of isophthalic acid included in the acid components of Samples 1 to 4.

D203C_S248C had lower decomposition efficiency than LCC at 50°C and 60°C. Whereas, both A172C_A209C and A172C_A209C_D203C_S248C had higher decomposition efficiency than LCC.

[Table 3]

| Sample 1 | Esterase | Reaction temperature (°C) | Upper row : Recovery volume ($\mu$mol) | | |
|---|---|---|---|---|---|
| | | | Lower row : Increase rate (%) | | |
| | | | Terephthalic acid | Isophthalic acid | All components |
| Test example 1 - 1 | Enzyme A | 50 | 5.78 + 59.5 | - - | 9.28 + 69.8 |
| Test example 1-2 | Enzyme B | | 4.74 + 30.8 | - - | 7.30 + 33.6 |
| Test example 1-3 | Enzyme C | | 2.79 - 23.1 | - - | 4.14 - 24.3 |
| Test example 1-4 | Enzyme D | | 3.63 | - - | 5.47 |

[Table 4]

| Sample 1 | Esterase | Reaction temperature (°C) | Upper row : Recovery volume ($\mu$mol) | | |
|---|---|---|---|---|---|
| | | | Lower row : Increase rate (%) | | |
| | | | Terephthalic acid | Isophthalic acid | All components |
| Test example 2-1 | Enzyme A | 60 | 17.4 + 22.6 | - - | 26.5 + 28.4 |
| Test example 2-2 | Enzyme B | | 18.3 + 29.5 | - - | 28.7 + 38.9 |
| Test example 2-3 | Enzyme C | | 12.4 - 12.5 | - - | 17.4 - 16.0 |
| Test example 2-4 | Enzyme D | | 14.2 - | - - | 20.7 - |

[Table 5]

| Sample 2 | Esterase | Reaction temperature (°C) | Upper row : Recovery volume ($\mu$mol) | | |
|---|---|---|---|---|---|
| | | | Lower row : Increase rate (%) | | |
| | | | Terephthalic acid | Isophthalic acid | All components |
| Test example 3-1 | Enzyme A | 50 | 6. 16 + 62. 7 | 0. 229 + 144 | 10.4 + 76. 7 |
| Test example 3-2 | Enzyme B | | 5.08 + 34. 1 | 0. 189 + 101 | 8.22 + 40.2 |
| Test example 3-3 | Enzyme C | | 2. 88 - 24.0 | 0 - 100 | 4. 32 - 24.3 |
| Test example 3-4 | Enzyme D | | 3.79 - | 0. 0938 - | 5. 86 - 26.4 |

[Table 6]

| Sample 2 | Esterase | Reaction temperature (°C) | Upper row : Recovery volume ($\mu$mol) | | |
|---|---|---|---|---|---|
| | | | Lower row : Increase rate (%) | | |
| | | | Terephthalic acid | Isophthalic acid | All components |
| Test example 4-1 | Enzyme A | 60 | 20. 6 + 34.5 | 0. 856 + 35.6 | 26. 5 + 49.5 |
| Test example 4-2 | Enzyme B | | 20. 1 + 31.2 | 0. 861 + 36.4 | 28. 7 + 41. 5 |
| Test example 4-3 | Enzyme C | | 12.6 - 17.8 | 0.494 - 21.7 | 17. 4 - 21.0 |
| Test example 4-4 | Enzyme D | | 15. 3 - | 0.632 - | 20.7 |

[Table 7]

| Sample 3 | Esterase | Reaction temperature (°C) | Upper row : Recovery volume ($\mu$mol) | | |
|---|---|---|---|---|---|
| | | | Lower row : Increase rate (%) | | |
| | | | Terephthalic acid | Isophthalic acid | All components |
| Test example 5-1 | Enzyme A | 50 | 6. 67 + 89. 1 | 0. 229 - 7.78 | 11.8 + 105 |
| Test example 5-2 | Enzyme B | | 5.58 + 58.0 | 0. 458 + 84. 5 | 9. 28 + 61. 6 |
| Test example 5-3 | Enzyme C | | 2. 90 - 17. 9 | 0. 192 - 22.5 | 4.60 - 19. 9 |
| Test example 5-4 | Enzyme D | | 3.53 - | 0.248 - | 5. 75 - |

[Table 8]

| Sample 3 | Esterase | Reaction temperature (°C) | Upper row : Recovery volume ($\mu$mol) | | |
|---|---|---|---|---|---|
| | | | Lower row : Increase rate (%) | | |
| | | | Terephthalic acid | Isophthalic acid | All components |
| Test example 6-1 | Enzyme A | 60 | 18. 0 + 26.8 | 1.56 + 36.0 | 29.0 + 32. 4 |
| Test example 6-2 | Enzyme B | | 18.8 + 32.0 | 1. 59 + 38. 7 | 31. 6 + 44.0 |
| Test example 6-3 | Enzyme C | | 12. 1 - 14.8 | 0.956 - 16.6 | 18. 1 - 17.4 |
| Test example 6-4 | Enzyme D | | 14.2 - | 1.15 - | 21.9 - |

[Table 9]

| Sample 4 | Esterase | Reaction temperature (°C) | Upper row : Recovery volume (μmol) | | |
|---|---|---|---|---|---|
| | | | Lower row : Increase rate (%) | | |
| | | | Terephthalic acid | Isophthalic acid | All components |
| Test example 7-1 | Enzyme A | 50 | 4.13 + 84.3 | 1.12 + 107 | 8.14 + 104 |
| Test example 7-2 | Enzyme B | | 3.88 + 73.1 | 1.02 + 87.8 | 7.42 + 85.5 |
| Test example 7-3 | Enzyme C | | 2.10 - 6.41 | 0.501 - 7.69 | 3.71 - 7.2 |
| Test example 7-4 | Enzyme D | | 2.24 | 0.543 | 4.00 |

[Table 10]

| Sample 4 | Esterase | Reaction temperature (°C) | Upper row : Recovery volume (μmol) | | |
|---|---|---|---|---|---|
| | | | Lower row : Increase rate (%) | | |
| | | | Terephthalic acid | Isophthalic acid | All components |
| Test example 8-1 | Enzyme A | 60 | 7.83 + 26.6 | 2.21 + 33.9 | 13.29 + 31.5 |
| Test example 8-2 | Enzyme B | | 11.1 + 79.2 | 3.03 + 83.8 | 19.4 + 93.7 |
| Test example 8-3 | Enzyme C | | 5.90 - 4.53 | 1.56 - 5.61 | 9.46 - 5.59 |
| Test example 8-4 | Enzyme D | | 6.18 | 1.65 | 10.0 |

(Test examples 9 to 12)

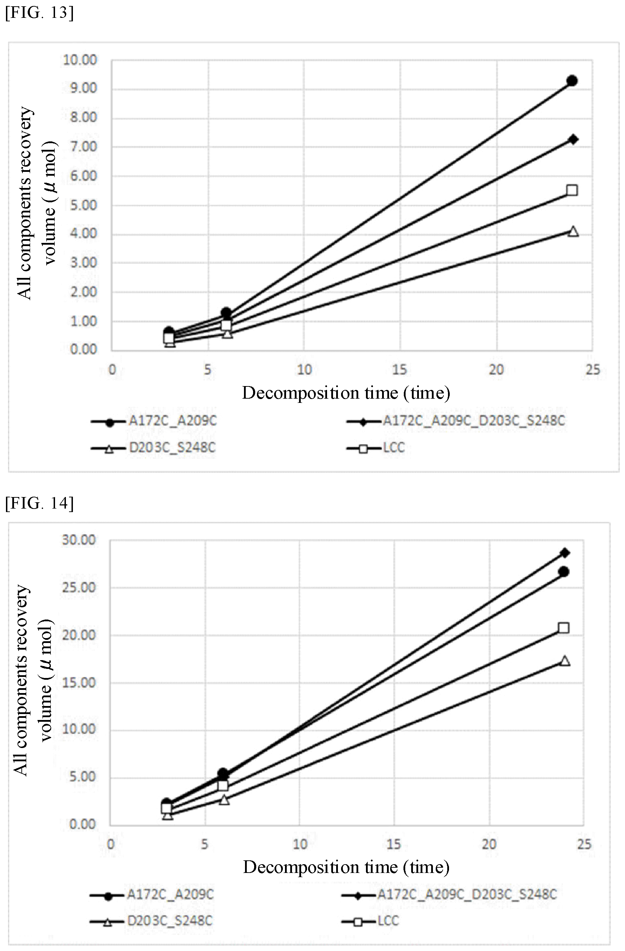

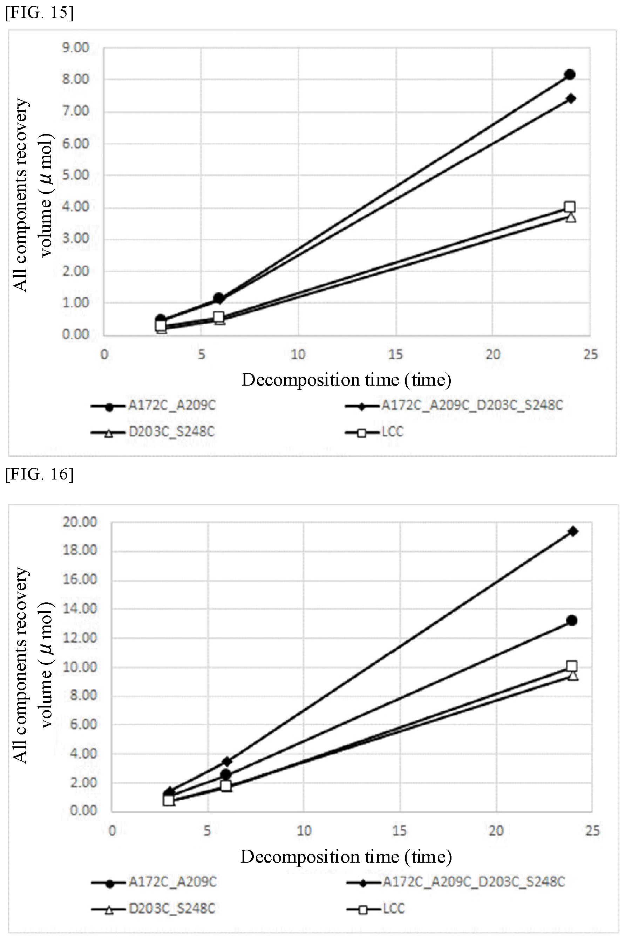

The decompsition rates of the esterases shown in Table 1 were compared using the polyesters of Samples 1 and 4 shown in Table 2 as the decomposition target. For each esterase, the recovery volume of the decomposition product at the decomposition time of 3 hours, 6 hours, and 24 hours was measured. The measurement results are shown in Table 11 to Table 14. Figure 13 shows transition of the recovery volume of the decomposition product when Sample 1 was decomposed at 50°C. Figure 14 shows transition of the recovery volume of the decomposition product when Sample 1 was decomposed at 60°C. Figure 15 shows transitoin of the recovery volume of the decomposition product when Sample 4 was decomposed at 50°C. Figure 16 shows transition of the recovery volume of the decomposition product when Sample 4 was decomposed at 60°C.

Regardless of the composition of the polyester, decomposition was faster with the enzyme A (A172C_A209C) and the enzyme B (A172C_A209C_D203C_S248C) than the enzyme C (D203C_S248C) and the enzyme D (LCC).

[Table 11]

| Sample 1 | Esterase | Reaction temperature (°C) | Recovery volume (μmol) | | |
|---|---|---|---|---|---|
| | | | 3hr | 6hr | 24hr |
| Test example 9-1 | Enzyme A | 50 | 0.59 | 1.24 | 9.28 |
| Test example 9-2 | Enzyme B | | 0.50 | 1.05 | 7.30 |
| Test example 9-3 | Enzyme C | | 0.28 | 0.60 | 4.14 |
| Test example 9-4 | Enzyme D | | 0.40 | 0.84 | 5.47 |

[Table 12]

| Sample 1 | Esterase | Reaction temperature (°C) | Recovery volume ($\mu$mol) | | |
|---|---|---|---|---|---|
| | | | 3hr | 6hr | 24hr |
| Test example 10-1 | Enzyme A | 60 | 2. 27 | 5. 38 | 26. 55 |
| Test example 10-2 | Enzyme B | | 2.10 | 5.15 | 28.72 |
| Test example 10-3 | Enzyme C | | 1. 14 | 2. 76 | 17.36 |
| Test example 10-4 | Enzyme D | | 1. 67 | 4.02 | 20.68 |

[Table 13]

| Sample 4 | Esterase | Reaction temperature (°C) | Recovery volume ($\mu$mol) | | |
|---|---|---|---|---|---|
| | | | 3hr | 6hr | 24hr |
| Test example 11-1 | Enzyme A | 50 | 0.48 | 1. 14 | 8.14 |
| Test example 11-2 | Enzyme B | | 0.48 | 1. 12 | 7. 42 |
| Test example 11-3 | Enzyme C | | 0.21 | 0.50 | 3.71 |
| Test example 11-4 | Enzyme D | | 0.27 | 0.56 | 4.00 |

[Table 14]

| Sample 4 | Esterase | Reaction temperature (°C) | Recovery volume ($\mu$mol) | | |
|---|---|---|---|---|---|
| | | | 3hr | 6hr | 24hr |
| Test example 12-1 | Enzyme A | 60 | 1.08 | 2.55 | 13.18 |
| Test example 12-2 | Enzyme B | | 1.40 | 3.50 | 19.41 |
| Test example 12-3 | Enzyme C | | 0.73 | 1.75 | 9.46 |
| Test example 12-4 | Enzyme D | | 0.69 | 1.70 | 10.02 |

(Test examples 13 to 14)

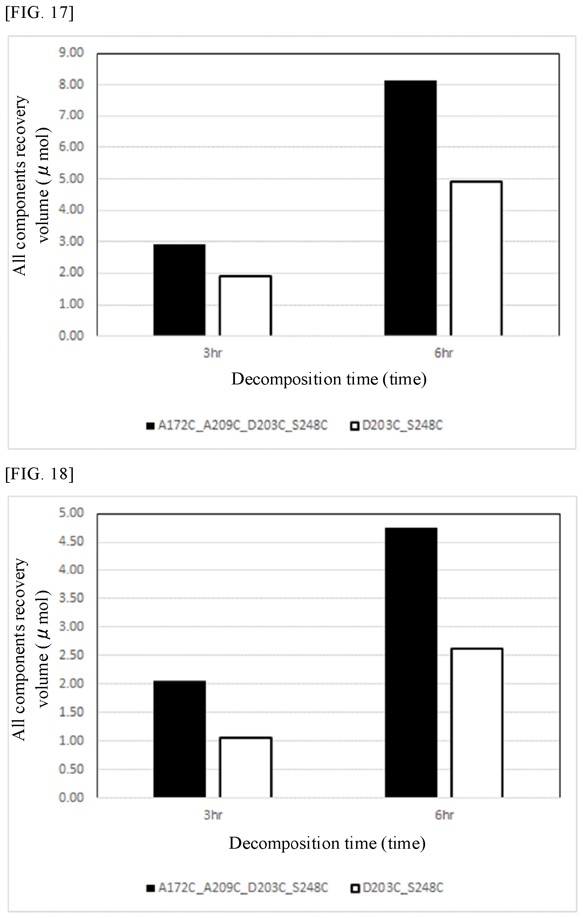

A decomposition test at 65°C was carried out with the enzyme B and the enzyme C shown in Table 1 using the polyesters of Samples 1 and 4 shown in Table 2 as the decomposition target. The measurement results are shown in Tables 15 to Table 16. Figure 17 shows the recovery volume of the decomposition product when Sample 1 was decomposed at 65°C. Figure 18 shows the recovery volume of the decomposition product when Sample 4 was decomposed at 65°C.

The decomposition rate was higher with the enzyme B in a high temperature region of 65°C. Moreover, it was found that the enzyme B has heat resistance at 65°C. Accordingly, it was found that the polyester can be decomposed at a higher temperature in which crystallization of the polyester is suppressed.

[Table 15]

| Sample 1 | Esterase | Reaction temperature (°C) | Recovery volume ($\mu$mol) | |
|---|---|---|---|---|
| | | | 3hr | 6hr |
| Test example 13-1 | Enzyme B | 65 | 2.92 | 8.15 |
| Test example 13-2 | Enzyme C | | 1.89 | 4.91 |

[Table 16]

| Sample 4 | Esterase | Reaction temperature (°C) | Recovery volume ($\mu$mol) | |
|---|---|---|---|---|
| | | | 3hr | 6hr |
| Test example 14-1 | Enzyme B | 65 | 2.07 | 4.75 |
| Test example 14-2 | Enzyme C | | 1.05 | 2.61 |

Although the description of the test result is omitted, when Sample 1 was heated for 48 hours at 70°C, the crystallinity increased to 5.4%. The higher the crystallinity of the polyester, the slower the decomposition rate by the esterase. Therefore, it is considered that the decomposition temperature is preferably 65°C or lower, and more preferably 60°C or lower.

(Test example 15)

[Preparation of Recycled polyester 1]

In a reaction container equipped with a stirrer, a distillation tube and a pressure reducing apparatus, 17.3 g in total of terephthalic acid and isophthalic acid (derived from a recycled material) collected under the condition of Test example 8-2, 21.3 g of terephthalic acid (derived from petroleum), 4.7 g of isophthalic acid (derived from petroleum), 66.2 g of bis-2-hydroxyethyl terephthalate (derived from a recycled material), and 8.1 g of ethylene glycol (derived from petroleum) were prepared, and heated to 250°C under nitrogen flow. It was maintained for 3 hours, allowing esterification to occur. Subsequently, 54 ppm of orthophosphoric acid and 120 ppm of germanium dioxide were added as a polymerization catalyst and stirred. Then, while the pressure was reduced to 133 Pa or lower in 1 hour, the inner temperature was increased from 250°C to 285°C, the mixture was stirred under a high vacuum of 133 Pa or lower until the predetermined viscosity was reached, and a polycondensation reaction was performed. The acquired polymer was extruded into water in strands and cut into pellets. As described above, the recycled polyester 1 was prepared.
The intrinsic viscosity and the color space Lab were measured for the acquired recycled polyester 1 as follows.

<Measurement of Intrinsic viscosity>

0.5000 g $\pm$ 0.001 g of a sample was dissolved to 50 mL of a mixed solvent of phenol:tetrachloroethane = 60:40 (mass ratio). An automatic viscosity measuring apparatus (manufactured by SUN Electronic Industries Corporation, ALC-6C) equipped with an Ubbelohde viscosity tube was used to measure the intrinsic viscosity (IV) at 20°C, and the intrinsic viscosity (IV) was 0.604 dl/g.

<Measurement of Color space Lab>

A color meter (manufactured by NIPPON DENSHOKU INDUSTRIES CO., LTD., ZE6000) was used to measure the color space Lab of the acquired recycled polyester 1, and the results were as follows: L=65.9, a=-1.0, and b=3.9.

(Test example 16)

[Preparation of Recycled polyester 2]

A recycled polyester 2 was prepared in the same manner as Test example 15, except that terephthalic acid and isophthalic acid (derived from a recycled material) collected under the condition of Test example 8-2 were not used, and 33.3 g of terephthalic acid (derived from petroleum), 10.0 g of isophthalic acid (derived from petroleum), 66.2 g of bis-2-hydroxyethyl terephthalate (derived from a recycled material), and 8.1 g of ethylene glycol (derived from petroleum) were used in Test example 15.
The intrinsic viscosity and the color space Lab of the acquired recycled polyester 2 were measured in the same manner as described above, and the results were as follows: the intrinsic viscosity (IV) = 0.612 dl/g, L=65.0, a=-1.2, and b=3.7.

(Test example 17)

[Preparation of Recycled polyester 3]

A recycled polyester 3 was prepared in the same manner as Test example 15, except that 66.2 g of bis-2-hydroxyethyl terephthalate derived from petroleum was used instead of 66.2 g of bis-2-hydroxyethyl terephthalate derived from a recycled material in Test example 15.

The intrinsic viscosity and the color space Lab of the acquired recycled polyester 3 were measured in the same manner as described above, and the results were as follows: the intrinsic viscosity (IV) = 0.610 dl/g, L=64.8, a=-1.1, and b=4.0.

(Test example 18)

[Preparation of Recycled polyester 4]

A recycled polyester 4 was prepared in the same manner as Test example 15, except that 43.3 g in total of terephthalic acid and isophthalic acid (derived from a recycled material) collected under the condition of Test example 8-2, 66.2 g of bis-2-hydroxyethyl terephthalate (derived from a recycled material), and 8.1 g of ethylene glycol (derived from petroleum) were used in Test example 15.

The intrinsic viscosity and the color space Lab of the acquired recycled polyester 4 were measured in the same manner as described above, and the results were as follows: the intrinsic viscosity (IV) = 0.602 dl/g, L=65.5, a=-1.1, and b=3.9.

(Test example 19)

[Preparation of Recycled polyester 5]

A recycled polyester 5 was prepared in the same manner as Test example 15, except that 32.5 g in total of terephthalic acid and isophthalic acid (derived form a recycled material) collected under the condition of Test example 8-2, and 82.8 g of bis-2-hydroxyethyl terephthalate (derived from a recycled material) were used in Test example 15.

The intrinsic viscosity and the color space Lab of the acquired recycled polyester 5 were measured in the same manner as described above, and the results were as follows: the intrinsic viscosity (IV) = 0.618 dl/g, L=66.0, a=-0.9, and b=3.4.

(Test example 20)

[Preparation of Recycled polyester 6]

In a reaction container equipped with a stirrer, a distillation tube and a pressure reducing apparatus, 86.6 g in total of terephthalic acid and isophthalic acid (derived from a recycled material) collected under the condition of Test example 8-2 and 40.5 g of ethylene glycol (derived from petroleum) were prepared, pressurized to 0.2 MPa with nitrogen, and maintained for 8 hours, allowing esterification to occur. Then, the pressure was released to atmospheric pressure, and the generated water was distilled off. Steps following the polymerization step were performed in the same manner as in Test example 15 to prepare a recycled polyester 6.

The intrinsic viscosity and the color space Lab of the acquired recycled polyester 6 were measured in the same manner as described above, and the results were as follows: the intrinsic viscosity (IV) = 0.620 dl/g, L=63.8, a=-1.3, and b=4.5.

(Test example 21)

[Preparation of Virgin polyester]

A virgin polyester was prepared in the same manner as Test example 15, except that terephthalic acid and isophthalic acid (derived from a recycled material) collected under the condition of Test example 8-2 were not used, and 33.3 g of terephthalic acid, 10.0 g of isophthalic acid, 66.2 g of bis-2-hydroxyethyl terephthalate, and 8.1 g of ethylene glycol, all derived from petroleum, were used in Test example 15.

The intrinsic viscosity and the color space Lab of the acquired virgin polyester were measured in the same manner as described above, and the results were as follows: the intrinsic viscosity (IV) = 0.609 dl/g, L=64.7, a=-1.2, and b=4.2.

From the results of Test examples 15 to 21, it was found that the recycled polyesters prepared with the polyester decomposition product (Test example 8-2) by the esterase of the present disclosure of Test example 15 and Test examples 17 to 19 had the same quality as the recycled polyester produced without using the polyester decomposition product (Test example 8-2) by the esterase of the present disclosure of Test example 16, and the virgin polyester of Test example 21.

The esterase, the polyester decomposition product, the method for treating the polyester, the method for producing the polyester, the polynucleotide, the vector, and the host cell of the present disclosure are described based on the above-described embodiments and examples; however, the present disclosure is not limited to the above-described embodiments and examples and may encompass various transformations, modifications, and improvements made to the various disclosed elements (including elements disclosed in the Claims, Description, and Drawings) within the scope of the present invention and based on the fundamental technical idea of the present invention. Moreover, various combinations, substitutions, and selections of the various disclosed elements are possible within the scope of the Claims of the present invention.

Further issues, objectives, and embodiments (including modifications) of the present invention are revealed also from the entire disclosure of the present invention including the Claims.

The numerical ranges disclosed herein are to be construed in such a manner that arbitrary numerical values and ranges falling within the disclosed ranges are treated as being concretely described herein, even where not specifically stated.

Some or all of the above-described embodiments may be described as in the following additional features, but are not limited to the following descriptions. Each additional feature can also be combined with each claim described in the Claims.

[Additional feature 1]

A polyester produced by the method for producing the polyester of the present disclosure.

[Additional feature 2]

A method for producing a monomer and/or an oligomer of the polyester by the method for treating the polyester of the present disclosure.

[Additional feature 3]

A monomer and/or an oligomer of the polyester prodused by the method for treating the polyester of the present disclosure.

Industrial Applicability

According to the esterase of the present disclosure, containers, films, sheets, fibers, and clothing produced by the decomposable polyester can be decomposed to a compound level. By using the polyester decomposition product as a raw material, a resource circulating recycling system of polyesters can be established.

## Claims

1.    An esterase, comprising wherein the esterase comprises at least 80% sequence identity with an amino acid sequence shown in SEQ ID NO: 1, and wherein A172 and A209 in the amino acid sequence are substituted.

2.    The esterase according to claim 1, wherein A172 and A209 are substituted with a cysteine residue (C).

3.    The esterase according to claim 1 or 2, wherein D203 and S248 in the amino acid sequence are further substituted.

4.    The esterase according to claim 3, wherein D203 and S248 are substituted with a cysteine residue (C).

5.    The esterase according to claim 3 or 4, wherein D203C and S248C form a disulfide bond.

6.    A method for treating a polyester, the method comprising a first step of decomposing a polyester by using the esterase according to any one of claims 1 to 5.

7.    The method for treating a polyester according to claim 6, wherein, in the first step, the polyester is decomposed in a treatment solvent having a temperature of 40°C or greater and a temperature lower than the glass transition temperature of the polyester within a range of pH 7 to 10.

8. The method for treating a polyester according to claim 7, wherein, in the first step, the polyester is decomposed in the treatment solvent having a temperature of 65°C or lower.

9. The method for treating a polyester according to any one of claims 6 to 8, wherein the polyester includes 50 mol% or greater of a terephthalic acid component with respect to the total amount of an acid component, and 65 mol% or greater of an ethylene glycol component with respect to the total amount of an alcohol component.

10. The method for treating a polyester according to claim 9, wherein the polyester further comprises 2 mol% or greater of an isophthalic acid component with respect to the total amount the acid component.

11. The method for treating a polyester according to any one of claims 6 to 10, wherein the polyester is at least either of a post-consumer product and a pre-consumer product.

12. The method for treating a polyester according to any one of claims 6 to 11, wherein the method further comprises a second step of isolating at least one compound selected from a group consisting of terephthalic acid, mono(2-hydroxyethyl) terephthalate, bis(2-hydroxyethyl) terephthalate, and salts thereof from the decomposition product acquired in the first step.

13. The method for treating a polyester according to claim 12, wherein, in the second step, at least one compound selected from a group consisting of isophthalic acid, mono(2-hydroxyethyl) isophthalate, bis(2-hydroxyethyl) isophthalate, and salts thereof is isolated together.

14. The method for treating a polyester according to any one of claims 6 to 13, wherein the method further comprises a third step of isolating a decomposition product derived from the alcohol component of the polyester from the decomposition product acquired in the first step.

15. A method for producing a polyester, the method comprising a fourth step of producing a polyester using a compound isolated in the method for treating a polyester according to any one of claims 12 to 14 as at least a part of a monomer.

16. The method for producing a polyester according to claim 15, wherein the compound comprises at least one compound selected from a group consisting of terephthalic acid, mono(2-hydroxyethyl) terephthalate, and bis(2-hydroxyethyl) terephthalate in the fourth step.

17. The method for producing a polyester according to claim 15 or 16, wherein the compound further comprises at least one compound selected from a group consisting of isophthalic acid, mono(2-hydroxyethyl) isophthalate, and bis(2-hydroxyethyl) isophthalate in the fourth step.

18. The method for producing a polyester according to any one of claims 15 to 17, wherein the compund comprises ethylene glycol in the fourth step.

19. A polyester decomposition product, wherein the polyester is decomposed by the esterase according to any one of claims 1 to 5.

20. A method for producing a polyester by using the polyester decomposition product according to claim 19.

21. A polynucleotide encoding the esterase according to any one of claims 1 to 5.

22. A vector comprising the polynucleotide according to claim 21.

23. A host cell to which the polynucleotide according to claim 21 is introduced.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

[FIG. 18]

## INTERNATIONAL SEARCH REPORT

| International application No. |
|---|
| **PCT/JP2024/029558** |

### A. CLASSIFICATION OF SUBJECT MATTER

*C12N 15/55*(2006.01)i; *C08J 11/24*(2006.01)i; *C08J 11/26*(2006.01)i; *C12N 1/15*(2006.01)i; *C12N 1/19*(2006.01)i; *C12N 1/21*(2006.01)i; *C12N 5/10*(2006.01)i; *C12N 9/16*(2006.01)i; *C12N 15/63*(2006.01)i; *C12P 7/18*(2006.01)i; *C12P 7/40*(2006.01)i; *C12P 7/62*(2022.01)i

FI: C12N15/55; C08J11/24; C12P7/62; C12P7/18; C12N15/63 Z; C12P7/40; C08J11/26; C12N1/19; C12N1/15; C12N9/16 ZNA; C12N5/10; C12N1/21

According to International Patent Classification (IPC) or to both national classification and IPC

### B. FIELDS SEARCHED

Minimum documentation searched (classification system followed by classification symbols)

C12N15/55; C08J11/24; C08J11/26; C12N1/15; C12N1/19; C12N1/21; C12N5/10; C12N9/16; C12N15/63; C12P7/18; C12P7/40; C12P7/62

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| X | JP 2019-520833 A (CARBIOS) 25 July 2019 (2019-07-25) claims 1-2, 9-11, 14-15, paragraphs [0040], [0052], [0057]-[0058], [0127]-[0128], [0130] - [0131], [0147], [0156]-[0163] | 1-9, 11-12, 14-16, 18-23 |
| Y | | 10, 13, 17 |
| Y | WO 2017/209223 A1 (SUNTORY HOLDINGS LTD.) 07 December 2017 (2017-12-07) paragraph [0003] | 10, 13, 17 |
| Y | JP 2022-547338 A (RITTEC UMWELTTECHNIK GMBH) 11 November 2022 (2022-11-11) paragraph [0004] | 10, 13, 17 |
| A | TOURNIER, V. et al. An engineered PET depolymerase to break down and recycle plastic bottles. Nature. 08 April 2020, vol. 580, pp. 216-219, DOI: 10.1038/s41586-020-2149-4 entire text, all drawings | 1-23 |

✓ Further documents are listed in the continuation of Box C.  ✓ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **18 September 2024** | **01 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2024/029558** |

C.    DOCUMENTS CONSIDERED TO BE RELEVANT

| Category* | Citation of document, with indication, where appropriate, of the relevant passages | Relevant to claim No. |
| --- | --- | --- |
| A | WO 2012/099018 A1 (AMANO ENZYME INC.) 26 July 2012 (2012-07-26) entire text, all drawings | 1-23 |

Form PCT/ISA/210 (second sheet) (July 2022)

## INTERNATIONAL SEARCH REPORT

| International application No. |
|---|
| **PCT/JP2024/029558** |

**Box No. I       Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

    ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| JP | 2019-520833 | A | 25 July 2019 | US 2019/0233803 A1 claims 16, 18, 27-30, 32-34, paragraphs [0044], [0056], [0061]-[0062], [0131]-[0132], [0134]-[0135], [0153], [0168]-[0174] WO 2018/011284 A1 EP 3485007 A1 CN 109563494 A KR 10-2019-0028753 A | | | |
| WO | 2017/209223 | A1 | 07 December 2017 | US 2019/0135975 A1 paragraph [0004] EP 3467004 A1 AU 2017275297 A1 CN 109196020 A KR 10-2019-0015241 A | | | |
| JP | 2022-547338 | A | 11 November 2022 | US 2023/0407001 A1 paragraph [0004] WO 2022/033735 A1 EP 3973015 A1 DE 102020123772 A1 KR 10-2022-0143138 A CN 115551931 A | | | |
| WO | 2012/099018 | A1 | 26 July 2012 | (Family: none) | | | |

Form PCT/ISA/210 (patent family annex) (July 2022)

**EP 4 772 639 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023139358 A
- WO 2012099018 A

- JP 2019520833 W

**Non-patent literature cited in the description**

- **TURNIER et al.** An engineered PET depolymerase to break down and recycle plastic bottles. *Nature*, 2020, vol. 580, 216-219

- **SHOSUKE YOSHIDA et al.** A bacterium that degrades and assimilates poly(ethylene terephthalate). *Sciencemag.org*, 2016, vol. 351, 1196